Europäisches Patentamt

European Patent Office

Office européen des brevets

(19)

(11) Veröffentlichungsnummer: **0 393 651**
**A1**

(12) **EUROPÄISCHE PATENTANMELDUNG**

(21) Anmeldenummer: **90107403.9**

(22) Anmeldetag: **19.04.90**

(51) Int. Cl.⁵: **C12M 1/10**

(30) Priorität: **20.04.89 DE 8904976 U**

(43) Veröffentlichungstag der Anmeldung:
**24.10.90 Patentblatt  90/43**

(84) Benannte Vertragsstaaten:
**AT BE DE DK FR GB NL**

(71) Anmelder: **Forschungszentrum Jülich GmbH**
**Postfach 1913**
**D-5170 Jülich(DE)**

(72) Erfinder: **Reich-Walber, Michael**
**Lütticher Strasse 5**
**D-5100 Aachen(DE)**

(54) **Bioreaktor mit Feststoff- oder Schüttkörperbett in einem drehbaren horizontal zylindrischen Behälter.**

(57) Ein Bioreaktor für die Durchführung biotechnologischer Prozesse in einem Feststoff-oder Schüttkörperbett, das einen allgemein horizontalen um seine Achse drehbaren Behälter weitgehend ausfüllt, von dessen Innenwand in radialer Richtung Längsrippen vorspringen, ist zur Vermeidung von Kurzschlußströmungen mit Ringblechabschnitten versehen, die dem Zylindermantel folgend in Rotationsrichtung von den Rippen vorspringen und senkrecht zur Achse verlaufen. Der Winkel $\Omega$ zwischen der freien Kante der Ringblechabschnitte und ihrer Anschlußkante an der Rippe liegt vorzugsweise zwischen 75 und 90°. Zweckmäßig sind 2 bis 4 gleichmäßig über den Zylindermantel verteilte Rippen, deren Radialabmessung etwa 1/3 des Reaktordurchmessers beträgt und die mit Ringblechabschnitten versehen sind, deren Abstand zwischen 15 und 50 cm liegt.

FIG. 1

EP 0 393 651 A1

## Bioreaktor mit Feststoff- oder Schüttkörperbett in einem drehbaren horizontal zylindrischen Behälter

Die Erfindung bezieht sich auf einen Bioreaktor für die Durchführung biotechnologischer Prozesse in einem Feststoff- oder Schüttkörperbett mit einem allgemein horizontalen, mit Flüssigkeitszu- und -ablauf und ggf. Gaszufuhr und/oder -entnahmeeinrichtungen versehenen allgemein zylindrischen Behälter, der um seine Achse drehbar ist und von der Innenwand in radialer Richtung vorspringende Längsrippen aufweist.

Ein allgemein horizontaler drehbarer Festbettreaktor der vorstehend genannten Art ist aus der EP-A2-0 237 039 der Anmelderin bekannt. Sei solchen Bioreaktoren wird durch die Rotation für eine fortgesetzte Umwälzung des Schüttkörpers im Festbett gesorgt, die eine Gasabgabe erleichtert und Verstopfungsgefahren mindert, da ständig ein gewisser Abrieb des Bewuchses mit Mikroorganismen erfolgt.

Solche füllkörperhaltigen Bioreaktoren können nach innen zu vorspringende Längsrippen in radialer Richtung aufweisen, welche die Schüttkörperumwälzung verbessert.

Dabei wird allerdings je nach Füllungsgrad und Flüssigkeitsspiegel im Reaktor ein gewisser Bereich der Flüssigkeit schüttkörperfrei gehalten, in dem dann Kurzschlußströmungen auftreten.

Ziel der Erfindung ist es hier Abhilfe zu schaffen.

Der zu diesem Zweck erfindungsgemäß vorgesehene Bioreaktor der eingangs genannten Art ist gekennzeichnet durch Ringblechabschnitte, die dem Zylindermantel folgend in Rotationsrichtung von den Rippen vorspringen und senkrecht zur Achse verlaufen.

Zwar sind neben den radial nach innen vorspringenden Längsrippen auch sogenannte Ringbleche zur Unterteilung des Volumens eines allgemein horizontalen drehbaren Bioreaktors bekannt, mit denen Kurzschlußströmungen vermieden werden, jedoch existieren bei solchen Reaktoren mit Ringscheiben in der Schüttung vor und hinter den Ringscheiben Gebiete mit schlechter Durchströmung (Totwassergebiete)

Nachfolgend wird die Erfindung anhand der beigefügten Figuren näher erläutert; es zeigen schematisch

Figur 1 eine mit Ringblechabschnitten versehene Längsrippe in perspektivischer Darstellung (Teildarstellung) und

Figur 2 die Verhältnisse in einem mit radial stehenden Längsrippen versehenen Roto-Bioreaktor zu unterschiedlichen Zeiten in Schnitten senkrecht zur Achse.

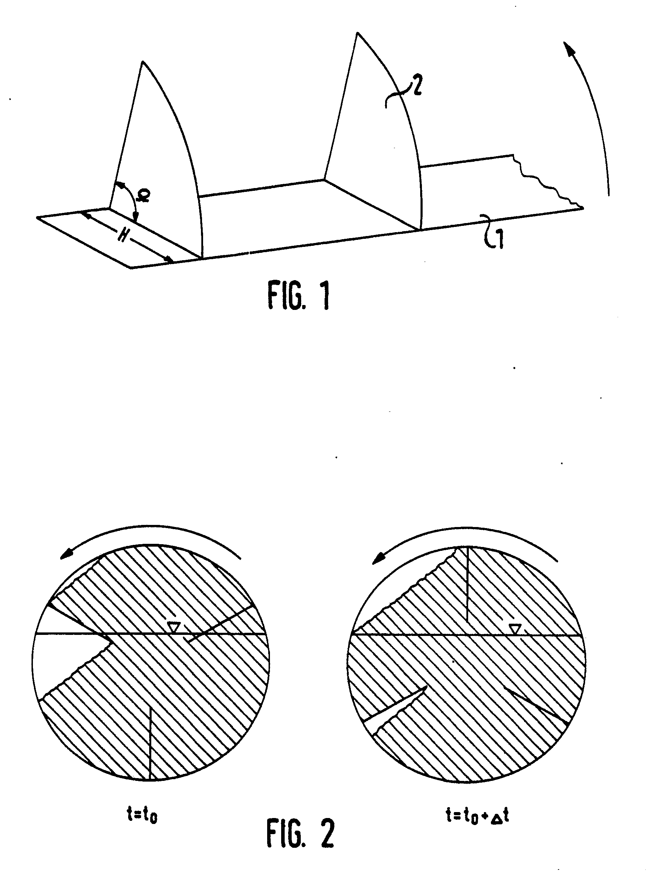

In der schematischen Schnittdarstellung von Figur 2 ist deutlich ersichtlich, daß im Verlaufe der Rotation bei Drehrohrreaktoren mit nach innen radial vorspringenden Längsrippen Phasen existieren, innerhalb derer ein mehr oder minder großes schüttkörperfreies Flüssigkeitsvolumen auftritt, das zu Kurzschlußströmungen Anlaß gibt.

Aus diesem Grunde sind, wie in Figur 1 schematisch dargestellt ist, auf die Rippe 1 Ringabschnitte 2 in Drehrichtung aufgesetzt, die für eine wiederkehrende Unterbrechung der Kurzschlußströmung sorgen.

Je nach Böschungswinkel ist der Winkel $\Omega$ zwischen der Anschlußkante und der freien Kante des Ringblechabschnitts zu wählen, der umso größer gewählt wird, je höher der Böschungswinkel des Schüttkörpers ist. Zweckmäßigerweise ist $\Omega$ zumindest gleich 50°, wobei Winkel zwischen etwa 75 und 90° bevorzugt werden.

Das Verhältnis von Reaktordurchmesser zu der Radialabmessung der im allgemeinen über die Gesamtlänge des Reaktors reichenden Rippen liegt insbesondere bei $\leq$ 6 : 1, und zwar vorzugsweise bei etwa 3 : 1.

Die Ringblechabschnitte 2 sind mit gleichmäßigem Abstand über die Rippe verteilt, wobei Abstände zwischen 15 und 50 cm bevorzugt werden. Zweckmäßigerweise liegen die Ringblechabschnitte aller Rippen jeweils in einer Ebene senkrecht zur Achse.

Obwohl die Zahl der radial stehenden Längsrippen nicht begrenzt ist, werden 2 bis 4 gleichmäßig über den Zylindermantel verteilte Rippen mit Ringblechabschnitten bevorzugt, wobei 3 Rippen besonders zweckmäßig sind.

Die vorstehend im wesentlichen bezogen auf einen Festbettdrehrohrreaktor mit biokatalysatorbelegter Schüttkörperfüllung, die $\geq$ 70 % des Reaktor-Innenraums einnimmt, beschriebene Erfindung ist selbstverständlich auch zusammen mit ggf. Kornmaterial enthaltenden breiartigen Massen (als Festbett) anwendbar, wie z.B. zur Intensivbehandlung kontaminierter Böden.

## Ansprüche

1. Bioreaktor für die Durchführung biotechnologischer Prozesse in einem Feststoff- oder Schüttkörperbett mit einem allgemein horizontalen, mit Flüssigkeitszu- und -ablauf und ggf. Gas-zufuhr- und/oder -entnahmeeinrichtungen versehenen, allgemein zylindrischen Behälter, der um seine Achse drehbar ist und von der Innenwand in radialer Richtung vorspringende Längsrippen aufweist, **gekennzeichnet durch** Ringblechabschnitte, die dem Zylindermantel folgend in Rotationsrichtung von den Rippen vorspringen und senkrecht zur Achse verlaufen.

2. Bioreaktor nach Anspruch 1,

**dadurch gekennzeichnet,**

daß der Winkel zwischen der freien Kante der Ringblechabschnitte und ihrer Anschlußkante an der Rippe zwischen 75 und 90° liegt.

3. Bioreaktor nach Anspruch 1 oder 2,

**gekennzeichnet durch**

zwei bis vier gleichmäßig über den Zylindermantel verteilte mit Ringblechabschnitten versehene Rippen.

4. Bioreaktor nach einem der vorangehenden Ansprüche,

**dadurch gekennzeichnet**

daß das Verhältnis von Reaktordurchmesser zu der Radialabmessung der Rippen ≦ 6 : 1, insbesondere 3 : 1 ist.

5. Bioreaktor nach einem der vorangehenden Ansprüche,

**dadurch gekennzeichnet**

, daß der Abstand der Ringblechabschnitte zwischen 15 und 50 cm liegt.

FIG. 1

t=t₀      FIG. 2      t=t₀+Δt

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | PATENT ABSTRACTS OF JAPAN vol. 9, np. 145 (C-287)(1868) 20 Juni 1985, & JP-A-60 27378 (IKEDA RIKA K.K.) 12 Februar 1985, * das ganze Dokument * | 1-5 | C12M1/10 |
| A | PATENT ABSTRACTS OF JAPAN vol. 8, no. 150 (C-233)(1587) 12 Juli 1984, & JP-A-59 55184 (TATSUJI KIKUCHI) 30 März 1984, * das ganze Dokument * | 1-5 | |

**RECHERCHIERTE SACHGEBIETE (Int. Cl.5)**

C12M

Der vorliegende Recherchenbericht wurde für alle Patentansprüche erstellt

| Recherchenort | Abschlußdatum der Recherche | Prüfer |
|---|---|---|
| DEN HAAG | 09 AUGUST 1990 | EPAILLARD P.J.H. |